Europäisches Patentamt

European Patent Office

Office européen des brevets

(19)

(11) Veröffentlichungsnummer: **0 081 732**
**B1**

(12)     # EUROPÄISCHE PATENTSCHRIFT

(45) Veröffentlichungstag der Patentschrift:
10.10.84

(21) Anmeldenummer: 82110992.3

(22) Anmeldetag: 27.11.82

(51) Int. Cl.³: **C 07 C 51/573**, C 07 C 53/12,
C 07 C 67/54, C 07 C 69/16

(54) Verfahren zur Abtrennung des Katalysatorsystems aus Reaktionsgemischen von Carbonylierungsreaktionen.

(30) Priorität: 11.12.81 DE 3149092

(43) Veröffentlichungstag der Anmeldung:
22.06.83 Patentblatt 83/25

(45) Bekanntmachung des Hinweises auf die Patenterteilung:
10.10.84 Patentblatt 84/41

(84) Benannte Vertragsstaaten:
AT BE CH DE FR GB IT LI NL

(56) Entgegenhaltungen:
EP - A - 0 056 488
DE - A - 1 202 778

(73) Patentinhaber: HOECHST AKTIENGESELLSCHAFT,
Postfach 80 03 20, D-6230 Frankfurt am Main 80 (DE)

(72) Erfinder: Erpenbach, Heinz, Dr., Oberbuschweg 22,
D-5000 Köln 50 (DE)
Erfinder: Gehrmann, Klaus, Dr., Geschwister Scholl
Strasse 31, D-5042 Erftstadt (DE)
Erfinder: Hörstermann, Peter, Dr.,
Theodor-Heuss-Strasse 2-4, D-5042 Erftstadt (DE)
Erfinder: Kübbeler, Hans-Klaus, Dr., Bünnagelring 31,
D-5357 Swisttal 8 (DE)
Erfinder: Kohl, Georg, von Geyr-Ring 61a, D-5030 Hürth
(DE)

**Beschreibung**

Die Erfindung betrifft ein Verfahren zur Abtrennung des Katalysatorsystems aus Reaktionsgemischen, die bei der Umsetzung von Methylacetat und/oder Dimethylether mit Kohlenmonoxid und gegebenenfalls Wasserstoff bei erhöhten Temperaturen zu Essigsäureanhydrid und gegebenenfalls Ethylidendiacetat in Gegenwart eines aus Carbonylkomplexen von Edelmetallen der Gruppe VIII des Periodensystems, Essigsäure, einer Organophosphor- oder Organostickstoff-Verbindung, Methyljodid und gegenenfalls Verbindungen carbonylbildender Nichtedelmetalle bestehenden Katalysatorsystems, z. B. gemäß DE-A-2 450 965, 2 836 084, 2 939 839 und 2 941 232, anfallen und unter einem Druck von 25 bis 150 bar und mit einer Temperatur von 100 bis 250° C die Reaktionszone verlassen.

Aus der DE-A-2 940 752 ist bereits ein Verfahren zur Abtrennung des Katalysators aus derartigen Reaktionsgemischen bekannt. Danach erfolgt die destillative Auftrennung des Reaktionsgemisches in flüchtige Produkte und Katalysatorsystem in einer Blitzdestillationskammer bei einem Druck von 3,8 bis 5,2 bar und Temperaturen von 110—130° C in Gegenwart eines Wasserstoffpartialdruckes von wenigstens 0,7 bar und eines Kohlenmonoxid-Partialdruckes von wenigstens 1 bar.

Die DE-A-3 013 257 beschreibt ein ähnliches Abtrennungsverfahren, wobei das Reaktionsprodukt durch Destillation bei 0,5 bar bis 1 bar in Gegenwart eines Kohlenmonoxid-Partialdruckes von mindestens 0,01 bar in flüchtige Bestandteile und Katalysatorsystem zerlegt wird.

Die DE-A-2 211 203 beschreibt schließlich ein Verfahren, nach dem man das unter Druck stehende Produktgemisch in einer Abtrennzone ohne Zufuhr von Wärme auf einen um mindestens 0,14 bar unter dem Reaktionsdruck liegenden Druck, vorzugsweise auf Normaldruck, entspannt, dabei die Carbonylierungsprodukte zum Teil verdampft und das zurückbleibende Reaktionsgemisch wieder dem Reaktor zuleitet.

Den genannten Verfahren liegt die Erfahrung zugrunde, daß die thermische Belastung des Reaktionsgemisches bei der destillativen Abtrennung der flüchtigen Carbonylierungsprodukte vom Katalysatorsystem zwecks Erhaltung der Katalysatoraktivität so gering wie möglich gehalten werden muß. Deshalb wird in der DE-A-2 211 203 die Verdampfung der flüchtigen Anteile durch adiabatische Entspannung ohne Wärmezufuhr durchgeführt. Dies ist bei der dort beschriebenen Herstellung von Essigsäure unschwer möglich. Bei größeren Anteilen höher als Essigsäure siedender Verbindungen, wie Essigsäureanhydrid oder Ethylidendiacetat, muß dieses Verfahren jedoch versagen, da der Wärmeinhalt des Reaktionsgemisches zur alleinigen Verdampfung der Reaktionsprodukte dann nicht mehr ausreicht. Eine äußere Zufuhr von Wärme ist daher zur weiteren destillativen Abtrennung der flüchtigen Anteile unerläßlich. Der negative Einfluß der zur Destillation notwendigen hohen Temperaturen kann verringert werden, wenn die Abtrennung in Gegenwart von Kohlenmonoxid und ggf. Wasserstoff erfolgt, wie aus den DE-A-2 940 752 und 3 013 257 hervorgeht. Beide Verfahren arbeiten in der Aufarbeitungsstufe bei Drucken von 0,5 bis 5,2 bar und führen den gesamten Produktstrom aus dem Reaktor direkt in den Verdampfer, wodurch sich eine starke Temperaturbelastung des Katalysators ergibt.

Das Ziel vorliegender Erfindung ist es daher, aus Reaktionsgemischen von Carbonylierungsreaktionen der geschilderten Art den Katalysator unter Bedingungen abzutrennen, die seine Zersetzung und Inaktivierung verhindern, so daß er ohne Reaktivierungsverluste zum Reaktor zurückgeführt werden kann.

Dieses Ziel wird dadurch erreicht, daß man das die Reaktionszone verlassende Reaktionsgemisch in einem auf 60 bis 140° C beheizten Abscheider auf einen Druck von 0,5 bis 3,5 bar entspannt, wobei die Hauptmenge der flüchtigen Anteile spontan verdampft; daß man aus dem den Abscheider verlassenden Flüssigkeitsstrom in einer Destillationszone bei einem Druck von 0,05—1 bar und einer Sumpftemperatur von 70—170° C den überwiegenden Teil der noch flüchtigen Anteile abdestilliert; und daß man die als Sumpfprodukt verbleibende Katalysatorlösung zur Reaktionszone zurückführt.

Das Verfahren der Erfindung kann außerdem wahlweise und bevorzugt dadurch gekennzeichnet sein, daß

a)   man die destillative Trennung in der Destillationszone in Gegenwart von Kohlenmonoxid und gegebenenfalls Wasserstoff durchführt;

b)   man die im Abscheider und in der Destillationszone abgetrennten flüchtigen Anteile vereinigt und gemeinsam destillativ aufarbeitet;

c)   die aus dem beheizten Abscheider verdampfenden flüchtigen Anteile einen Nebelabscheider durchströmen, der das Mitreißen von Flüssigkeitstropfen verhindert;

d)   der Nebelabscheider Filtergewebe aus korrosionsfesten Stoffen, vorzugsweise aus Glasfasern oder Edelstahl, enthält.

Das die Reaktionszone unter einem Druck von 25 bis 150 bar verlassende Reaktionsgemisch weist bevorzugt eine Temperatur von 150 bis 200° C auf und wird in einem vorzugsweise auf 70 bis 130° C beheizten Abscheider auf einen Druck von vorzugsweise 0,8 bis 2 bar entspannt, wobei der größere Teil der unumgesetzten Ausgangsstoffe sowie der gebildeten Endprodukte spontan verdampft.

Auf diese Weise wird bereits unter schonenden Bedingungen ein großer Anteil der flüchtigen Reaktionsprodukte vom gelösten Katalysatorsystem getrennt. Der zurückbleibende Flüssigkeitsstrom wird anschließend in einer Kurzwegdestillation unter einem Druck von 0,05 bis 1 bar, vorzugsweise in Gegenwart von Kohlenmonoxid und gegebenenfalls Wasserstoff, vom größten Teil der noch enthaltenen flüchtigen Reaktionsprodukte befreit, woraufhin die als Sumpfprodukt verbleibende Katalysatorlösung zum Reaktor zurückgeführt wird. Das im Abscheider abgetrennte Dampfgemisch wird gemeinsam mit dem Kopfprodukt der Kurzwegdestillation der weiteren Aufarbeitung zugeführt. Durch diese Vorabtrennung der im Abscheider verdampfenden Produktanteile müssen nur noch 10 bis 40 Gew.-% der insgesamt zu verdampfenden Bestandteile im nachfolgenden Destillationsschritt abgetrennt werden, wodurch die thermische Belastung des Katalysators auf ein Minimum reduziert wird.

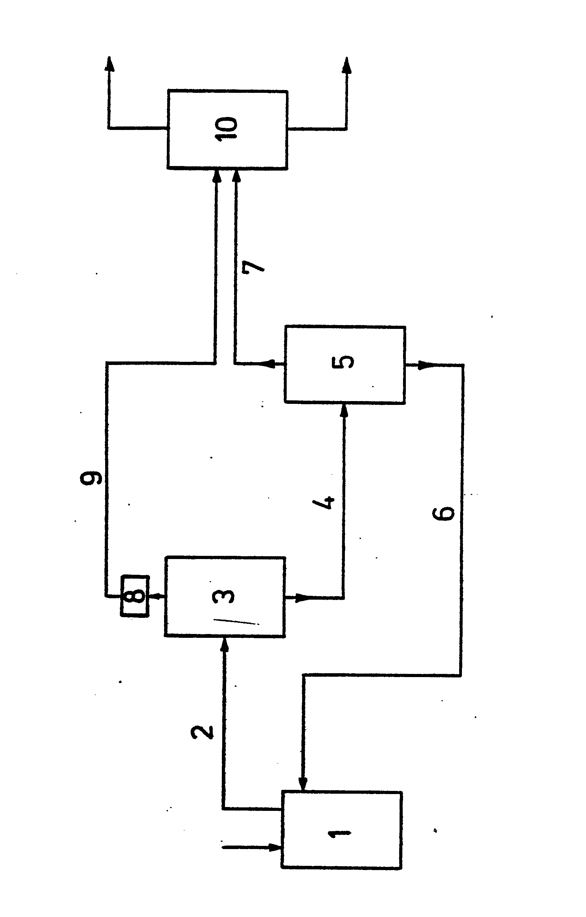

Die Erfindung wird im folgenden anhand der Zeichnung näher erläutert:

Das bei der Carbonylierung erhaltene Reaktionsgemisch wird aus dem Carbonylierungsreaktor 1 abgezogen und über Leitung 2 einem Abscheider 3 zugeführt. Der aus gelöstem Gas und Flüssigkeit bestehende Produktstrom steht bei Austritt aus dem Reaktor unter einem Druck von 25 bis 150 bar und besitzt eine Temperatur von 100 bis 250°C, vorzugsweise von 150 bis 200°C.

In dem auf 70—130°C beheizten Abscheider 3 wird der Produktstrom auf einen Druck von 0,5 bis 3,5 bar, vorzugsweise 0,8 bis 2 bar, entspannt und in einen flüssigen und einen dampfförmigen Anteil getrennt. Der flüssige Anteil gelangt über Leitung 4 in die Trennstufe 5, die aus einem Verdampfer, ggf. mit fünf nachgeschalteten Destillationsböden, besteht. Als Verdampfer kann ein Umlaufverdampfer zur geringeren Temperaturbelastung des Katalysators jedoch vorzugsweise ein Fallstrom- oder Dünnschichtverdampfer, eingesetzt werden. Dieser wird entweder unter Normaldruck (1013 mbar) bei einer Sumpftemperatur von 134°C oder aber unter verringertem Druck bei entsprechend tieferer Temperatur, beispielsweise bei 150 mbar/96°C, betrieben. Um Aktivitätseinbußen des Katalysators zu vermeiden, wird der Sumpf des Verdampfers 5 mit stündlich 1 bis 100 l, vorzugsweise 10 bis 25 l, Kohlenmonoxid oder ggf. eines Gemisches aus Kohlenmonoxid und Wasserstoff beschickt. Der flüssige Destillationsrückstand, der das gelöste Katalysatorsystem enthält, wird über Leitung 6 zum Reaktor 1 zurückgepumpt. Das Destillat des Verdampfers 5, bestehend aus überwiegend Essigsäureanhydrid und Essigsäure neben Ethylidendiacetat, Methyljodid und nichtumgesetztem Methylacetat, gelangt über Leitung 7 gemeinsam mit den über einen Nebelabscheider 8, der mit Filtergewebe aus Glasfasern oder Hastelloy B gefüllt ist, und über Leitung 9 aus dem Abscheider 3 abgezogenen Dampfanteilen zur weiteren, nicht erfindungsgemäßen destillativen Aufarbeitung 10.

## Beispiel 1

Dem Carbonylierungsreaktor 1 werden unter einem Druck von 75 bar und bei einer Temperatur von 180°C 5841 g/h Reaktionsprodukt entnommen und dem Abscheider 3 zugeführt. Hier werden bei einer Wandtemperatur von 75°C und einem Druck von 1,1 bar 2829 g/h dampfförmige Anteile (67 Gew.-% der insgesamt verdampften Produkte), bestehend aus 25,14 Gew.-% Essigsäureanhydrid, 11,35 Gew.-% Essigsäure, 0,24 Gew.-% Ethylidendiacetat, 37,63 Gew.-% Methylacetat, 22,99 Gew.-% Methyljodid, 0,42 Gew.-% Aceton, 1,8 Gew.-% Kohlenmonoxid, 0,13 Gew.-% Kohlendioxid, 0,03 Gew.-% Methan und 0,27 Gew.-% Stickstoff, von den flüssigen Bestandteilen abgetrennt. Die Dampfanteile gelangen über den Nebelabscheider 8 in die erste Trennstufe der nicht erfindungsgemäßen nachfolgenden Aufarbeitung 10. Die flüssigen Anteile, 3012 g/h, werden in einen Fallstromverdampfer 5 mit nachgeschalteten fünf Destillationsböden überführt. Die Abtrennung der flüchtigen Bestandteile erfolgt dort bei 1 bar und einer Sumpftemperatur von 134°C, wobei der Sumpf mit 16 l/h Synthesegas (CO : $H_2$ 1 : 1) beschickt wird. 1388 g/h Destillat (33 Gew.-% der insgesamt verdampften Produkte) mit einer Zusammensetzung von 57,2 Gew.-% Essigsäureanhydrid, 22,6 Gew.-% Essigsäure, 16,7 Gew.-% Methylacetat und 3,5 Gew.-% Methyljodid gelangen ebenfalls in die erste Trennstufe der weiteren destillativen Aufarbeitung 10. Im Verdampfer 5 fallen als Sumpfprodukt 1624 g/h Katalysatorlösung mit 23,4 Gew.-% Essigsäureanhydrid, 6,15 Gew.-% Essigsäure, 0,38 Gew.-% Ethylidendiacetat und 70,07 Gew.-% Katalysatorsystem (Rh-Komplex und Methyltributylphosphoniumjodid) an und werden zum Reaktor 1 zurückgeführt.

## Beispiel 2

Dem Carbonylierungsreaktor 1 werden unter einem Druck von 70 bar bei 184°C 23 450 g/h Reaktionsprodukt entnommen und dem Abscheider 3 zugeführt. Hier werden bei einer Wandtemperatur von 125°C und einem Druck von 1,4 bar 14 750 g/h dampfförmige Anteile (89 Gew.-% der insgesamt verdampften Produkte), bestehend aus 19,42 Gew.-% Essigsäureanhydrid, 13,6 Gew.-% Essigsäure, 0,11 Gew.-% Ethylidendiacetat, 42,51 Gew.-% Methylacetat, 23,32 Gew.-% Methyljodid, 0,56 Gew.-% Aceton, 0,47 Gew.-% Kohlenmonoxid, 0,02 Gew.-% Kohlendioxid, 0,01 Gew.-% Methan und 0,09 Gew.-% Stickstoff, von den flüssigen Anteilen abgetrennt. Die flüssigen Anteile (8700 g/h) werden einem Fallstromverdampfer 5 mit fünf Destillationsböden zugeführt. Hier erfolgt die Abtrennung der flüchtigen Bestandteile bei einem Druck von 150 mbar und einer Sumpftemperatur von 96°C, wobei der Sumpf mit 18 l/h Synthesegas (CO/ $H_2$ = 1 : 1) angeperlt wird. Es werden 1800 g/h Destillat (11 Gew.-% der insgesamt verdampften Produkte) mit einer Zusammensetzung von 36 Gew.-% Essigsäureanhydrid, 35 Gew.-% Essigsäure, 22,8 Gew.-% Methylacetat und 6,2 Gew.-% Methyljodid erhalten. Das Destillat gelangt gemeinsam mit den über den Nebelabscheider 8 abgezogenen dampfförmigen Anteilen des Abscheiders 3 zur ersten Trennstufe der nicht erfindungsgemäßen Aufarbeitung 10. Als Sumpfprodukt fallen im Verdampfer 56 900 g/h Katalysatorlösung mit 18 Gew.-% Essigsäureanhydrid, 11,68 Gew.-% Essigsäure, 0,12 Gew.-% Ethylidendiacetat und 70,2 Gew.-% Katalysatorsystem (Rh-Komplex und Methyltributylphosphoniumjodid) an, die zum Reaktor 1 zurückgeführt werden.

Beispiel 3

Dem Carbonylierungsreaktor 1 werden unter einem Druck von 90 bar bei 186°C 4676 g/h Reaktonsprodukt entnommen und dem Abscheider 3 zugeführt. Hier werden bei einer Wandtemperatur von 110°C und einem Druck von 0,9 bar 2212 g/h dampfförmige Anteile (68 Gew.-% der insgesamt verdampften Produkte), bestehend aus 22,24 Gew.-% Essigsäureanhydrid, 12,52 Gew.-% Essigsäure, 5,83 Gew.-% Ethylidendiacetat, 36,12 Gew.-% Methylacetat, 22,06 Gew.-% Methyljodid, 0,61 Gew.-% Kohlenmonoxid, 0,49 Gew.-% Methan und 0,13 Gew.-% Wasserstoff, von den flüssigen Bestandteilen abgetrennt. Die flüssigen Anteile (2464 g/h) werden einem Dünnschichtverdampfer 5 ohne zusätzliche Destillationsböden zugeführt. Die Abtrennung der flüchtigen Bestandteile erfolgt dort bei 100 mbar und einer Sumpftemperatur von 85°C, wobei der Sumpf mit 16 l/h Kohlenmonoxid angeperlt wird. 1055 g/h Destillat (32 Gew.-% der insgesamt verdampften Produkte) mit einer Zusammensetzung von 26,45 Gew.-% Essigsäureanhydrid, 47,78 Gew.-% Essigsäure, 12,23 Gew.-% Ethylidendiacetat, 9 Gew.-% Methylacetat und 4,55 Gew.-% Methyljodid gelangen gemeinsam mit dem über den Nebelabscheider 8 abgezogenen Dampfgemisch des Abscheiders 3 zur weiteren nicht erfindungsgemäßen Aufarbeitung 10. Als Sumpfprodukt des Verdampfers 5 werden 1409 g/h Katalysatorlösung mit 20,23 Gew.-% Essigsäureanhydrid, 13,84 Gew.-% Ethylidendiacetat, 5,32 Gew.-% Essigsäure und 60,61 Gew.-% Katalysatorsystem (Rh-Komplex und Methyltributylphosphoniumjodid) zum Reaktor 1 zurückgeführt.

In allen aufgeführten Beispielen sind selbst nach Laufzeiten von mehr als 6 Monaten keine Aktivitätseinbußen des Katalysatorsystems zu beobachten.

## Patentansprüche

1. Verfahren zur Abtrennung des Katalysatorsystems aus Reaktionsgemischen, die bei der Umsetzung von Methylacetat und/oder Dimethylether mit Kohlenmonoxid und gegebenenfalls Wasserstoff bei erhöhten Temperaturen zu Essigsäureanhydrid und gegebenenfalls Ethylidendiacetat in Gegenwart eines aus Carbonylkomplexen von Edelmetallen der Gruppe VIII des Periodensystems, Essigsäure, einer Organophosphor- oder Organostickstoff-Verbindung, Methyljodid und gegebenenfalls Verbindungen carbonylbildender Nichtedelmetalle bestehenden Katalysatorsystems anfallen und unter einem Druck von 25 bis 150 bar und mit einer Temperatur von 100 bis 250°C die Reaktionszone verlassen, dadurch gekennzeichnet, daß man das die Reaktionszone verlassende Reaktionsgemisch in einem auf 60 bis 140°C beheizten Abscheider auf einen Druck von 0,5 bis 3,5 bar entspannt, wobei die Hauptmenge der flüchtigen Anteile spontan verdampft; daß man aus dem den Abscheider verlassenden Flüssigkeitsstrom in einer Destillationszone bei einem Druck von 0,05—1 bar und einer Sumpftemperatur von 70 bis 170°C den überwiegenden Teil der noch flüchtigen Anteile abdestilliert; und daß man die als Sumpfprodukt verbleibende Katalysatorlösung zur Reaktionszone zurückführt.

2. Verfahren nach Anspruch 1, dadurch gekennzeichnet, daß man die destillative Trennung in der Destillationszone in Gegenwart von Kohlenmonoxid und gegebenenfalls Wasserstoff durchführt.

3. Verfahren nach Anspruch 1 oder 2, dadurch gekennzeichnet, daß man die im Abscheider und in der Destillationszone abgetrennten flüchtigen Anteile vereinigt und gemeinsam destillativ aufarbeitet.

4. Verfahren nach einem der Ansprüche 1—3, dadurch gekennzeichnet, daß die aus dem beheizten Abscheider verdampfenden flüchtigen Anteile einen Nebelabscheider durchströmen, der das Mitreißen von Flüssigkeitstropfen verhindert.

5. Verfahren nach Anspruch 4, dadurch gekennzeichnet, daß der Nebelabscheider Filtergewebe aus korrosionsfesten Stoffen, vorzugsweise aus Glasfasern oder Edelstahl, enthält.

## Claims

1. Process for separating the catalyst system from reaction mixtures which are obtained by reacting methyl acetate and/or dimethylether with carbon monoxide and optionally hydrogen at elevated temperatures to acetic anhydride and optionally ethylidene diacetate in the presence of a catalyst system consisting of carbonyl complexes of noble metals of group VIII of the Periodic System, acetic acid, an organophosphorus or organonitrogen compound, methyl iodide and optionally compounds of carbonyl yielding common metals, and which issue from the reaction zone under a pressure of 25 to 150 bars and at a temperature of 100 to 250°C, which comprises: releasing the reaction mixture coming from the reaction zone in a separator heated to 60 to 140°C to a pressure of 0,5 to 3,5 bars with spontaneous evaporation of the bulk of volatile constituents; distillatively separating in a distilling zone under a pressure of 0,05 to 1 bar at a base temperature of 70 to 170°C the bulk of still volatile constituents from the stream of liquid matter leaving the separator; and recycling the catalyst solution retained as base product to the reaction zone.

2. Process as claimed in claim 1, wherein the distillative separation in the distilling zone is effected in the presence of carbon monoxide and optionally hydrogen.

3. Process as claimed in claim 1 or 2, wherein the volatile constituents separated in the separator and distilling zone, respectively, are combined and jointly worked up distillatively.

4. Process as claimed in any of claims 1 to 3, wherein the volatile constituents evaporated in the heated separator are passed through a mist separator preventing liquid droplets from being carried along.

5. Process as claimed in claim 4, wherein the mist separator contains filter gauzes of corrosionproof materials, preferably of glass fibers or stainless steel.

## Revendications

1. Procédé de séparation du système catalytique des mélanges réactionnels obtenus par réaction de l'acétate de méthyle et/ou de l'éther diméthylique avec le monoxyde de carbone et, éventuellement, l'hydrogène à des températures élevées en anhydride acétique et, éventuellement, en diacétate d'éthylidène en présence d'un système catalytique constitué par des complexes carbonyles de métaux nobles du groupe VIII de la classification périodique, de l'acide acétique, un composé organophosphoré ou organoazoté, de l'iodure de méthyle et, éventuellement, des composés de métaux non nobles formant des carbonyles, et sortant de la zone de réaction sous une pression de 25—150 bars et à une température de 150—250°C, caractérisé en ce que l'on détend à une pression de 0,5—3,5 bars dans un séparateur chauffé à 60—140°C le mélange de réaction sortant de la zone de réaction avec évaporation spontanée de la majeure partie des constituants volatils; on sépare par distillation dans une zone de distillation, sous une pression de 0,05—1 bar et à une température de bas de colonne de 70—170°C la majeure partie des constituants encore volatils du courant liquide sortant du séparateur et on recycle dans la zone de réaction la solution catalytique restant comme produit de bas de colonne.

2. Procédé selon la revendication 1, caractérisé en ce que l'on effectue la séparation par distillation dans la zone de distillation en présence de monoxyde de carbone et, éventuellement, d'hydrogène.

3. Procédé selon la revendication 1 ou 2, caractérisé en ce que l'on réunit les constituants volatils séparés dans le séparateur et la zone de distillation et on les soumet en commun au traitement complémentaire par distillation.

4. Procédé selon l'une des revendications 1 à 3, caractérisé en ce que l'on fait passer les constituants volatils évaporés dans le séparateur chauffé dans un séparateur de brouillard empêchant l'entraînement de gouttes liquides.

5. Procédé selon la revendication 4, caractérisé en ce que le séparateur de brouillard contient des tissus filtrants en matériaux résistants à la corrosion, de préférence en fibres de verre ou en acier spécial.